# EUROPÄISCHE PATENTANMELDUNG

(11) **EP 1 544 619 A2**
(43) Veröffentlichungstag der Anmeldung: **22.06.2005**
(21) Anmeldenummer: 04027030.8
(22) Anmeldetag: 13.11.2004
(51) Int. Cl.: G01N 33/68, C12Q 1/54, G01N 33/569

(54) **Trockenchemischer Test**

(30) Priorität: 19.12.2003 DE 10359716
(71) Anmelder: Analyticon Biotechnologies AG, 35104 Lichtenfels (DE)
(72) Erfinder: Meisegeier, Bernhard, Dr., 97209 Veitshöchheim (DE); Siekmann, Werner, Dr., 35104 Lichtenfels (DE)

(57) **Zusammenfassung**

1. Trockenchemischer Test
2. Die Erfindung betrifft ein langzeitstabilisiertes Mittel zum Nachweis von Bestandteilen in Flüssigkeiten, bestehend aus: flächenförmigemTräger, katalytisch aktiven und/oder katalytisch nichtaktiven Eiweißkörpern und monomeren oder peptidartig-verküpften Aminocarbonsäuren und Polyhydroxyverbindungen. Der Träger weist Langzeitstabilität auf.

## Beschreibung

### Anwendung der Erfindung

Die Erfindung betrifft ein Verfahren zur Langzeitstabilisierung trockenchemischer Schnelltests, die zur Erfassung von Analyten in Flüssigkeiten eingesetzt werden, vorzugsweise von Tests, die katalytisch aktive Proteine und/oder immunologisch aktive biologische Proteine enthalten.

Diese Tests, die vereinfachte Analysensysteme darstellen, können in medizinischen und veterinärmedizinischen Arztpraxen, in medizinischen Laboratorien, in der Klinik, in der Lebensmittelanalytik, Umweltanalytik, im Militärwesen und in anderen Bereichen angewendet werden. Die Tests werden sowohl im Routinelabor als auch dezentral z.B. am Krankenbett, in der Sprechstunde, auf der Station eingesetzt.

Die Tests liefern in kürzester Zeit, auf sehr einfache Weise, bei einfacher Handhabung und sehr geringem Arbeitsaufwand ein Ergebnis.

### Charakteristik des Standes der Technik

Trockenchemische Schnelltests werden seit längerer Zeit (D. Kutter, Schnelltests in der klinischen Diagnostik, Urban & Schwarzenberg, München, Wien, Baltimore 1983; L. J. Bride, Urinanalysis and Body Fluids, Lippincott, Philadelphia, New York 1997) zum qualitativen Nachweis oder zur halbquantitativen Bestimmung von Analyten in Körperflüssigkeiten eingesetzt. So werden Teststreifen in der Harnanalyse, zur Bestimmung von Analyten im Blut, zum Erfassen von Mikroorganismen oder in der Umweltanalytik verwendet. Biosensoren und chromatographische Schnelltests dienen zum Schnellnachweis unterschiedlichster Analyten.

Die spezifische Erfassung vieler Analyten gelingt nur mittels enzymatischer oder immunchemischer Nachweisverfahren (C. A. Burtis, E. R. Ashwood Clinical Chemistry, W. B. Saunders Company, Philadelphia, London, Toronto, Montreal, Sydney, Tokyo, 1994; L. Thomas, Labor und Diagnose, TH-Books Verlagsgesellschaft mbH, Frankfurt/Main, 1998). Enzyme und immunologisch aktive Proteine unterliegen während der Lagerung Änderungen der nativen Konformation, diese Denaturierungsvorgänge führen allmählich zur Abnahme der enzymatischen bzw. immunologischen Aktivität. Teste, die Enzyme oder immunologisch aktive Proteine als Reaktionspartner haben, würden permanete Aktivitätseinbußen und Empfindlichkeitsverminderungen zeigen, wenn keine ausreichende Stabilisierung vorgenommen würde. Im großen Umfang werden biochemische und mikrobiologische und verschiedene chemische Analyten wie Oligo- und Polypeptide, Proteine, Antigene, Antikörper, Infektionserreger, Hormone spezifisch mit immunologischen Reaktionen nachgewiesen (D. M. Kemeny, A practical guide to ELISA, Pergamon Press, 1991).

Die Stabilisierung der auf den festen Träger aufgebrachten immunologisch aktiven Verbindungen stellt bei der Herstellung lager- und versandfähiger Teste ein besonderes Problem dar.

Der Nachweis von Glucose im Harn hat für die Erkennung des Diabetes mellitus und für Verlaufskontrollen große Bedeutung. Tests zum Nachweis von Glucose basieren fast ausschließlich auf enzymatischer Reaktion. Meist bestehen diese Teste aus einem saugfähigen Träger, der mit Glucoseoxidase, Peroxidase, einem Indikator und Puffer getränkt ist. Der Erhalt der enzymatischen Aktivität in der Zubereitung ist Voraussetzung für einen industriell standardisiert gefertigten, lager- und transportfähigen trockenchemischen Test.

### Ziel der Erfindung

Der vorliegenden Erfindung liegt die Aufgabe zugrunde, einen langzeitstabilisierten Teststreifen zum Nachweis von Analyten in Körperflüssigkeiten und ein Verfahren zur Langzeitstabilisierung an Festphasenmaterialien gebundener oder aufgebrachter enzymatischen Zubereitungen oder immunologisch reaktiver Verbindungen bereitzustellen.

### Darlegung des Wesens der Erfindung

Gegenstand der Erfindung ist ein verbesserter, langzeitstabilisierter Teststreifen zum Nachweis von Glucose in biologischen Flüssigkeiten und ein Verfahren zur Stabilisierung in saugfähigem Trägermaterial eingelagerter oder an Festphasenmaterialien gebundener katalytisch aktiver oder immunologisch aktiver Proteine. Als saugfähige Trägermaterial kommen alle Filterpapiere, Vliese oder Filze aus Cellulose, anorganischen oder organischen Fasern oder Polymeren infrage, als Festphasenmaterialien fungieren Kunststoffe wie Polystyrol, Polyethylen, Polypropylen, Polyvinylchlorid, Polyester, Polyvinylacetat, Polycarbonat, anorganische Materialien.

Es wurde überraschend gefunden, daß eine Kombination von aminogruppenhaltigen organischen Säuren der allgemeinen Zusammensetzung R-CH(NH₂)-(CH₂)ₙ-COOH in einer Konzentration von 0,0007 bis 0,7 mol/l, vorzugsweise 0,007 bis 0,07 mol/l, wobei R den Rest H oder eine Amino-, Imino-, Guanidino-, Hydroxy-, Thiol-, Hydroxyalkyl-, Aminoalkyl-, Carboxyalkyl-, aromatische hetrocyclische Gruppe repräsentieren kann und n den Wert von 0 bis 6, vorzugsweise 0 annehmen kann,
oder von höhermolekularen homodet oder heterodet peptidartig verknüpften Verbindungen dieser Aminocarbonsäuren in Konzentrationen von 0,006g/100ml bis 3,81g/100ml Stickstoff, vorzugsweise 0,06 bis 0,64 g Stickstoff/100ml,
oder von höhermolekularen peptidartig-verknüpften Verbindungen , die vorzugsweise durch technische oxidativ-thermische Abbauvorgänge aus großtechnisch gewonnenen depolymerisierten Hydrolyseprodukten fibrillärer Skleroproteine des tierischen Bindegewebes kollagenen Ursprungs, beispielsweise "Gelafusal" gewonnen wurden,
oder von einer Polypeptid-Lösung, die einen Stickstoffgehalt von 0,006g/100ml bis 1,3g/100ml, vorzugsweise 0,06 bis 0,6g/100ml,
mit Polyhydroxyverbindungen linearer Struktur oder cyclischer Struktur oder Polyhydroxycarbonylverbindungen, vorzugsweise acetalartig verknüpften Polyhydroxycarbonylverbindungen, z.B. alpha-D-Glucopyranosyl-beta-D-fructo-furanosid in Konzentrationen von 0,0003 bis 1,2 mol, vorzugsweise 0,01 bis 0,3 mol die Stabilität der Teste enorm erhöhen kann.

Besonders wurden Polyhydroxyverbindungen linearer Struktur mit der allgemeinen Struktur C₆H₁₄O₆ mit wahlweise unterschiedlicher geometrischer Isomerie der Hydroxygruppen aber auch Polyhydroxyverbindungen cyclischer Struktur der allgemeinen Struktur C₆H₁₂O₅oder C₆H₁₂O₆ wahlweise unterschiedlicher geometrischer Isomerie der Hydroxylgruppen bevorzugt.

### Ausführungsbeispiele

### Beispiel 1

Filterpapier (Schleicher und Schüll 2316) wird mit Lösung 1 getränkt, die sich pro 100 ml zusammensetzt aus:

| | |
|---|---|
| Citratpuffer, pH 5,5 | 80 ml |
| o-Tolidin-dihydrochlorid | 0,38 g |
| Tartrazin | 0,020 g |
| Peroxidase | 0,120 g |
| Glucoseoxidase | 0,210 g |
| Gelafusal (Fällung mit 3,75 g N) | 0,60 g |
| Polyhydroxyverbindung C₆H₁₄O₆ | 1,80 g |
| cyclische Polyhydroxyverbindung C₆H₁₂O₅ | 0,60 g |

mit Ethanol auf 100 ml auffüllen

Nach dem Trocknen bei 70 °C wird das Papier mit Lösung 2 getränkt, die 0,40 g Ethylcellulose, 0,10 g Poly(methylvinylether-alt-maleinsäure-anhydrid) in 70 ml Toluol und 30 ml Ethanol enthält.

Nach dem Trocknen wird das Papier in 5 x 5 mm große Stücke geschnitten und auf einem PVC-Träger aufgeklebt.

Zur Überprüfung der Stabilität wurden die Teststreifen in Aluminiumdosen in Gegenwart von Trockenmittel bei 50 °C unterschiedliche Zeiten gelagert. Zu den entsprechenden Meßpunkten wurden Teststreifen entnommen und in Urine mit unterschiedlichen Glucosegehalten getaucht. Nach 60 Sekunden Reaktionszeit wurden mit dem Urinteststreifenauswertegerät Combi-Scan der Analyticon Biotechnologies AG die Remissionswerte ermittelt. Folgende Tabelle zeigt die Ergebnisse im Vergleich zu einem kommerziell verfügbaren Urinteststreifen eines anderen Herstellers.

| Remissionswerte | | | | |
|---|---|---|---|---|
| Lagerung bei 50 °C | 0 mg Glucose/dl | 150 mg Glucose/dl | 500 mg Glucose/dl | 1500 mg Glucose/dl |
| Tag vor Lagerung | 65,2 | 34,8 | 17,6 | 11,7 |
| 2 Wochen | 67,5 | 34,2 | 15,9 | 11,3 |
| 3 Wochen | 68,7 | 35,6 | 16,1 | 10,9 |
| 4 Wochen | 68,8 | 35,2 | 15,8 | 10,8 |
| 3 Monate | 65,3 | 33,9 | 17,3 | 11,1 |
| 7 Monate | 67,9 | 32,7 | 17,1 | 11,2 |

| kommerzieller Vergleichstest | | | | |
|---|---|---|---|---|
| Lagerung bei 50 °C | 0 mg Glucose/dl | 150 mg Glucose/dl | 500 mg Glucose/dl | 1500 mg Glucose/dl |
| Tag vor Lagerung | 59,4 | 11,2 | 7,2 | 5,9 |
| 2 Wochen | 62,6 | 14,0 | 7,6 | 6,8 |
| 3 Wochen | 61,9 | 15,7 | 9,3 | 8,0 |
| 4 Wochen | 65,2 | 15,5 a) | 8,1 a) | 7,9 a) |

| | | | | |
|---|---|---|---|---|
| a) Teststreifen zeigt abweichende dunkelgrün-braune Verfärbungen, teilweise fleckig, nach einer Lagerzeit über 4 Wochen nicht mehr auswertbar | | | | |

### Beispiel 2

### Nachweis von Streptokokken A Antigen mittels Immunosensor:

Entsprechend einem Zwei-Elektroden-Layout wurden Silberpaste und Graphitpaste auf PVC-Folie (10 x 50 mm) mittels Siebdrucktechnik gedruckt. Dabei wurden zu 500 mg Graphitpaste 250 µl einer handelsüblichen Lösung von "Gelafusal" (Stickstoff-Gehalt 0,9 g/100 ml) gemischt. Die Trocknung erfolgte 5 Minuten bei 100 °C. Ebenso wurden die Elektrodenkontakte gedruckt und bei 100° C 5 Minuten getrocknet.

### Präparation des aktivierten anti-Streptokokken-A-Glucoseoxidase-Konjugates:

Für die Reaktion wurden 5 mg anti-Streptokokken-A-Glucoseoxidase-Konjugat in 1 ml MES-Puffer [2-(N-Morpholino-ethansulfonsäure; 0,1 mol/l/0,15 mol/l NaCl, pH 4,7] gelöst, 1 ml einer frisch hergestellten 10%igen (w/v) Lösung von Ethyl-dimethyl-amino-propyl)-carbodiimid in Wasser und 1 ml einer frisch hergestellten 14%igen (w/v) Lösung von N-Hydroxysuccinimid-sulfonsäure-Natriumsalz in Wasser und 4 ml MES-Puffer pipettiert. Mittels rotierender Bewegung wurde 15 Minuten inkubiert und gemischt. 5 µl dieser Mischung aktivierten anti-Streptokokken-A-Glucoseoxidase-Konjugates und 5 µl Kupplungspuffer [0,1 mol/l Natriumdihydrogenphosphat/Dinatriumhydrogenphosphat/0,15 mol/l Natriumchlorid-Lösung, pH 7,5] wurden auf die Arbeitselektrode pipettiert und 30 min einwirken gelassen. Man saugte ab, pipettierte 5 µl einer Lösung von Albumin aus dem Serum vom Rind (5,0 g/l) und 0,50 mol Polyhydroxyverbindung C₆H₁₄O₆ in Wasser und trocknete im Vakuum bei 30-50 °C über 60 Minuten.

Stromaufwärts über den Elektroden wurden 5 µl anti-Streptokokken-A-(Rabbit)-Peroxidase-Konjugat (1 mg/ml), in einer 0,3 mmolaren Lösung eines bei 80% Ammoniumsulfatsättigung aus dem Serum von Pferd gefälltes, in reinem Wasser löslichen Proteins, der 0,3 mol alpha-D-Glucopyranosyl-beta-D-fructofuranosid zugesetzt wurden, aufgetragen und im Vakuum bei 40 °C getrocknet.

Zur Montage des Immunosensors wurden das Flüssigkeitsaufnahmeelement (Faservlies Schleicher&Schüll 0966, 10 x 25 mm), das mit einer Lösung von 3 g C₂₀H₃₇NaO₇S in 100 ml gereinigten Wassers, getränkt und getrocknet wurde, und die Abdeckung (10 x 22 mm, Polyester, mit halbkreisförmigem Ausschnitt ) auf die Elektrodenplatte so geklebt wurde, daß sich ein Abstand in der Höhe des Flüssigkeitsaufnahmeelementes über der Elektrodenplatte und somit ein Reaktionsraum ergab.

### Prüfung:

Von zwei Patienten (Patient 1: Alter 7 Jahre, Geschlecht: männlich, Grund des Arztbesuches: Erkältungssymptome, Infekt der Luftwege; Patient 2: Alter 5 Jahre, Geschlecht: weiblich; Grund des Arztbesuches: Halsschmerzen) wurden je drei Rachenabstriche entnommen.

Abstrich 1 beider Patienten wurde jeweils in einem Extraktionsgefäß mit einer Lösung aus 200 µl Glycin (0,1 mol/l)/HCl, pH 2,5 und 200 µl Natriumnitrit (1 mol/l) zwei Minuten extrahiert und mit 200 µl Trispuffer (0,2 mol/l, pH 9) versetzt. Je 10 µl dieser Extraktionslösung wurden auf die Probenaufgabestelle des Sensors pipettiert. Nach einer Reaktionszeit von 20 Sekunden wurden 30 µl einer Lösung pipettiert, die D-Glucose (20 mmol/l) und Kaliumjodid (3 mmol/l) in Wasser enthielt.

Ergebnis der Messung: Patient 1: negativ; Patient 2: positiv auf Streptokokken A Antigen, entspräche gemäß Eichkurve ca. 4 x 10⁵ KBE/ml (Koloniebildende Einheiten/ml).

Abstrich 2 wurde zwecks späterer Prüfung bei -70 °C gelagert

Abstrich 3 beider Patienten wurde zwecks kultureller Anzucht in Röhrchen mit Transportmedium transportiert.

Die Kultivierung erfolgte auf Schaedler-Blutagar über 24 Stunden bei 37 °C anaerob.

Auswertung qualitativ: Patient 1: negativ; Patient 2: positiv (Bestätigung der beta-hämolysierenden Streptokokken der serologischen Gruppe A mit Streptokokken-Identifizierungstest zum Nachweis betahämolysierender Streptokokken (Oxoid).

Koloniezählung (S. Isaac, D. Jennings: Kultur von Mikroorganismen, Spektrum Akademischer Verlag Heidelberg, Berlin, Oxford, S. 87): 2 x 10⁵ KBE/ml.

Prüfung des Sensors nach Lagerung über 4 Wochen bei 50 °C:

Zur Prüfung wurde Abstrich 2 verwendet.

Ergebnis der Messung: Patient 1: negativ; Patient 2: positiv auf Streptokokken A Antigen, entspräche gemäß Eichkurve ca. 3 x 10⁵ KBE/ml (Koloniebildende Einheiten/ml).

## Patentansprüche

1. Prüfmittel zur Erfassung von Analyten in einer flüssigen Probe, bestehend aus einem flächenförmigen Träger, **dadurch gekennzeichnet, daß** sich auf dem Träger spezifische Reaktanden befinden, die zur Langzeitstabilisierung in einer Kombination von aminogruppenhaltigen organischen Säuren bzw. von höhermolekularen, peptidartig verknüpften Verbindungen von Aminocarbonsäuren mit Polyhydroxyverbindungen eingebettet sind.

2. Prüfmittel gemäß Anspruch 1, **dadurch gekennzeichnet, daß** der Träger mit einer flüssigkeitsabsorbierenden Schicht versehen ist, die katalytisch aktive Eiweißkörper , monomere oder peptidartig verknüpfte Aminocarbonsäuren, Polyhydroxyverbindungen und ein Chromogen enthält und das Prüfmittel der Bestimmung von Glucose in Körperflüssigkeiten dient.

3. Prüfmittel gemäß Anspruch 1, **dadurch gekennzeichnet, daß** die entstehende Farbänderung, ein Fluoreszenzsignal oder ein elektochemisches Signal ausgewertet wird.

4. Prüfmittel gemäß Anspruch 1, **dadurch gekennzeichnet, daß** sich auf der Oberfläche des Trägers Reaktanden befinden, die aus katalytisch aktiven und/oder katalytisch nichtaktiven Eiweißkörpern bestehen.

5. Prüfmittel nach Anspruch 1, **dadurch gekennzeichnet, daß** die Aminocarbonsäuren die allgemeine Zusammensetzung R-CH(NH₂)-(CH₂)ₙ-COOH haben, wobei R den Rest H oder eine Amino-, Imino-, Guanidino-, Hydroxy-, Thiol-, Hydroxyalkyl-, Aminoalkyl-, Carboxyalkyl-, aromatische hetrocyclische Gruppe repräsentieren kann und n den Wert von 0 bis 6, vorzugsweise 0 annehmen kann und in einer Konzentration von 0,0007 bis 0,7 mol/l, vorzugsweise 0,007 bis 0,07 mol/l, eingesetzt werden.

6. Prüfmittel nach Anspruch 1, **dadurch gekennzeichnet, daß** die Aminocarbonsäuren höhermolekular und homodet oder heterodet peptidartig verknüpft sein können.

7. Prüfmittel nach Anspruch 6 , **dadurch gekennzeichnet, daß** die ein oder mehrere Polypeptide in Konzentrationen von 0,006g bis 3,81g Stickstoff /100ml, vorzugsweise 0,06 bis 0,64 g Stickstoff/100ml eingesetzt werden.

8. Prüfmittel nach Anspruch 1, **dadurch gekennzeichnet, daß** die höhermolekularen peptidartig verknüpften Aminocarbonsäuren auch durch technische oxidativ-thermische Abbauvorgänge aus großtechnisch gewonnenen depolymerisierten Hydrolyseprodukten fibrillärer Skleroproteine des tierischen Bindegewebes kollagenen Ursprungs gewonnen werden und vorzugsweise Gelafusal eingesetzt wird.

9. Prüfmittel nach Anspruch 1, **dadurch gekennzeichnet, daß** die Polyhydroxyverbindungen von linearer Struktur sind und die allgemeine Struktur C₆H₁₄O₆ mit wahlweise unterschiedlicher geometrischer Isomerie der Hydroxygruppen besitzen.

10. Prüfmittel nach Anspruch 1, **dadurch gekennzeichnet, daß** die Polyhydroxyverbindungen von cyclischer Struktur sind und die allgemeine Struktur C₆H₁₂O₅oder C₆H₁₂O₆ mit wahlweise unterschiedlicher geometrischer Isomerie der Hydroxygruppen besitzen.

11. Prüfmittel nach Anspruch 1, **dadurch gekennzeichnet, daß** die Polyhydroxycarbonylverbindungen acetalartig verknüpftet sind.

12. Prüfmittel und Verfahren nach Anspruch 9, 10 und 11, **dadurch gekennzeichnet, daß** die Polyhydroxycarbonylverbindungen in Konzentrationen von 0,0003 bis 1,2 mol/l, vorzugsweise 0,01 bis 0,3 mol/l eingesetzt werden.

13. Verfahren zur Herstellung eines stabilisierten Prüfmittels zur Erfassung von Analyten in einer flüssigen Probe, insbesondere nach den Ansprüchen 1-12, **dadurch gekennzeichnet, daß** der Träger mit einer wäßrigen Lösung, die katalytisch aktive und/oder katalytisch nichtaktive Eiweißkörper und eine Kombination von Aminocarbonsäuren bzw. von höhermolekularen, peptidartig verknüpften Verbindungen von Aminocarbonsäuren mit Polyhydroxyverbindungen enthält, behandelt und getrocknet wird.

14. Verfahren nach Anspruch 13, **dadurch gekennzeichnet, daß** der Träger mit einer flüssigkeitsabsorbierenden Schicht versehen ist, die katalytisch aktive Eiweißkörper, monomere oder peptidartig verknüpfte Aminocarbonsäuren, Polyhydroxyverbindungen und ein Chromogen enthält und unter Ausnutzung der Farbänderung nach Kontakt mit der Probe zur Bestimmung von Glucose in Körperflüssigkeiten dient.

15. Verfahren nach Anspruch 13, **dadurch gekennzeichnet, daß** sich auf dem Träger als Reaktanden katalytisch aktive und/oder katalytisch nicht aktive Eiweißkörper befinden, die in monomere oder peptidartig verknüpfte Aminocarbonsäuren und Polyhydroxyverbindungen eingebettet sind.
